# EUROPEAN PATENT APPLICATION

(11) **EP 4 610 226 A1**
(43) Date of publication of application: **03.09.2025**
(21) Application number: 24171633.1
(22) Date of filing: 22.04.2024
(51) Int. Cl.: C01B 33/193

(54) **SILICA PARTICLE AND PREPARATION METHOD THEREOF, AND ORAL CLEANING AND CARE COMPOSITION**

(30) Priority: 29.02.2024 CN 202410228184
(71) Applicant: Jinsanjiang (Zhaoqing) Silicon Material Co., LTD., Zhaoqing, Guangdong 526200 (CN)
(72) Inventor: ZHENG, Yixuan, Zhaoqing, Guangdong, 526200 (CN); HUANG, Dan, Zhaoqing, Guangdong, 526200 (CN); WANG, Xianwei, Zhaoqing, Guangdong, 526200 (CN); MI, Shuang, Zhaoqing, Guangdong, 526200 (CN); ZHENG, Peiwen, Zhaoqing, Guangdong, 526200 (CN)
(74) Representative: Porta & Consulenti Associati S.p.A.

(57) **Abstract**

A silica particle and a preparation method thereof, and an oral cleaning and care composition are disclosed. The silica particle provided by the present disclosure has (a particle size D₉₀- a particle size D₁₀)/ a particle size D₅₀=1.25-2.5, a particle size D₁₀<1 µm, a particle size D₃₀=1.5 µm-2 µm, a particle size D₅₀=3 µm-4 µm, a particle size D₇₅<5.5 µm, a particle size D₉₀=6 µm-8.5 µm, a particle size D₉₇<9 µm, and an apparent density of >0.3 g/mL. The silica particle provided by the present disclosure has a small particle size, which can effectively block dentinal tubules and microtubules on the tooth surface, isolate the channel of external stimulation and hinder the transmission of excitement to a dental pulp nerve, thus alleviating a tooth sensitivity. Without chemical reactions, the silica particle will not cause a secondary damage to sensitive teeth, is safe and effective, and has a long-lasting effect.

## Description

### TECHNICAL FIELD

The present disclosure relates to the technical field of oral care, and in particular to a silica particle and a preparation method thereof, and an oral cleaning and care composition.

### BACKGROUND

Dentin hypersensitivity, also known as tooth hypersensitivity, is a symptom of tooth soreness caused by external stimulation such as temperatures (cold, hot), chemicals (sour, sweet), mechanical actions (friction, biting hard objects), characterized by rapid onset, sharp pain and short duration, and is a relatively common oral problem. Currently, there are two methods to treat the dentin hypersensitivity: 1) remineralization with a strontium salt and fluoride blocks exposed dentinal tubules, reduces hydraulic conduction, and isolates a channel connected to the external stimulation, thus achieving the effect of alleviating and preventing tooth sensitivity, this method involves the occurrence of a chemical reaction (for example: the strontium salt enters the dentinal tubules and reacts with the calcium in the tooth to form calcified strontium apatite), which takes a relatively long time to take effect truly, and the chemical reaction occurred in the oral cavity causes an unknown risk for special groups such as pregnant women and children; and 2) a potassium salt, Chinese herbal medicine (such as peony phenol) and the like can play a role in relieving pain and paralyzing dental nerves to relieve the tooth sensitivity, which requires long-term use, and the tooth sensitivity problem may reappear once stopping.

Therefore, it is of great significance to develop an oral care product that can effectively relieve the tooth sensitivity, has a long-lasting effect, and is safe and environmentally friendly.

### SUMMARY

The objective of the present disclosure is to provide a silica particle and a preparation method thereof, and an oral cleaning and care composition.

The technical solution used in the present disclosure is as follows:
A silica particle, which has (a particle size D₉₀- a particle size D₁₀)/ a particle size D₅₀=1.25-2.5, a particle size D₁₀<1 µm, a particle size D₃₀=1.5 µm-2 µm, a particle size D₅₀=3 µm-4 µm, a particle size D₇₅<5.5 µm, a particle size D₉₀=6 µm-8.5 µm, a particle size D₉₇<9 µm, and an apparent density of >0.3 g/mL.

Preferably, the silica particle has a particle size D₁₀>0.5 µm.

Further preferably, the silica particle has a particle size D₁₀>0.7 µm.

Preferably, the silica particle has an apparent density of >0.4 g/mL. The silica particle having a high density can be deposited into exposed dentinal tubules more quickly during tooth brushing, and is less likely to fall off along tooth brushing, making the effect more lasting.

Preferably, the silica particle has a copper loss of 2 mg to 11 mg, a Radioactive Dentin Abrasivity (RDA) value <140, and a Pellicle Cleaning Ratio (PCR) value ≥ 100. If the copper loss and RDA value are small, a damage to tooth abrasion is little, and if the PCR is large, a cleaning ability is strong.

Further preferably, the silica particle has a copper loss of 4 mg-7 mg, a RDA value <135, and a PCR value d 100.

Preferably, a water absorption capacity of the silica particle is <20 mL/20 g.

Further preferably, the water absorption capacity of the silica particle is <15 mL/20g.

Preferably, a Loss on Ignition (1000 °C)of the silica particle is <8.5%.

Further preferably, the Loss on Ignition (1000 °C) of the silica particle is <6.5%.

Preferably, the silica particle is in an irregular shape.

A preparation method for the silica particle as described above includes the following steps:
1) dissolving polyethylene glycol in water, adding a sodium silicate solution and a sulfuric acid solution simultaneously at a maintained flow rate, and controlling a pH value of a reaction solution to 1-2 during reaction; stopping adding the sulfuric acid solution when an addition amount of the sodium silicate solution reaches 10 m³-15m³, and continuing to add the sodium silicate solution until a pH value of a reaction solution is 2-4, and performing aging to obtain acidic silica sol;
2) adding the acidic silica sol to a reaction tank, then adding a certain amount of sodium sulfate solution, heating to 50°C-60°C and starting stirring; adding the sodium silicate solution and the sulfuric acid solution simultaneously at a maintained flow rate, and controlling a pH value of a reaction solution to 7-9 and a temperature to 50 °C-60 °C during reaction, and reacting for 20 min-40 min;
3) stopping adding the sodium silicate solution and the sulfuric acid solution, heating to 60 °C-70 °C, adding a certain amount of sodium silicate solution, adding the sulfuric acid solution at a maintained flow rate until a pH value of a reaction solution is 5-7, and stopping adding the sulfuric acid solution for aging; and
4) adding a sulfuric acid solution with a high concentration at a maintained flow rate until a pH value of a reaction solution is 3-5, stopping adding the sulfuric acid solution, and performing press-filtering, washing, drying and crushing to obtain the silica particle.

Preferably, an addition ratio of the polyethylene glycol to the water in step 1) is 300 kg-400 kg: 25 m³-35 m³.

Preferably, the sodium silicate solution in step 1) has a concentration of 0.2 mol/L-1 mol/L.

Preferably, the sodium silicate solution in step 1) has a flow rate of 1.5 m³/h-2.5 m³/h.

Preferably, the sulfuric acid solution in step 1) has a concentration of 1 mol/L-2 mol/L.

Preferably, the aging in step 1) lasts for 5 h-7 h.

Preferably, the acidic silica sol in step 2) has a mass fraction of 1%-3%.

Preferably, the sodium sulfate solution in step 2) has a mass fraction of 15%-20%.

Preferably, the sodium silicate solution in step 2) has a concentration of 0.5 mol/L-1.25 mol/L.

Preferably, the sodium silicate solution in step 2) has a flow rate of 5 m³/h-12 m³/h.

Preferably, the sulfuric acid solution in step 2) has a concentration of 2 mol/L-6 mol/L.

Preferably, the acidic silica sol, the sodium sulfate solution, and the sodium silicate solution in step 2) has a volume ratio of 1: 10-30: 10-30.

Preferably, the sodium silicate solution in step 3) has a concentration of 0.5 mol/L-1.25 mol/L.

Preferably, the sodium silicate solution in step 3) has an addition amount of 8 m³-12 m³.

Preferably, the sulfuric acid solution in step 3) has a flow rate of 2 m³/h-8 m³/h.

Preferably, the aging in step 3) lasts for 10 min-45 min.

Preferably, the sulfuric acid solution with a high concentration in step 4) has a volume fraction of 75%-85%.

Preferably, the sulfuric acid solution with a high concentration in step 4) has a flow rate of 1 m³/h-4 m³/h.

An oral cleaning and care composition, comprising the silica particle as described above.

Preferably, the oral cleaning and care composition further comprises an orally acceptable excipient.

Preferably, the orally acceptable excipient is at least one of a moisturizer, a thickener, a surfactant, an abrasive, and an essence.

Preferably, the oral cleaning and care composition has a pH value of 6-10.

Preferably, the oral cleaning and care composition is a toothpaste.

In the present disclosure, the reaction is carried out by setting up a two-step method. Firstly, the sodium silicate solution and the sulfuric acid solution are added to a silica sol system at a certain speed, when silica crystal nuclei generate, the reaction conditions are changed and the temperature is raised to 60 °C-70 °C to make the reaction be performed in an alkaline environment. After the pH value reaches a specified value, the sulfuric acid solution with a high concentration is added to adjust the pH value at an end point, and the silica particle is finally obtained. The specific reaction mechanisms are as follows:
In step 2), the acidic silica sol and the sodium sulfate solution are added to the reaction tank (as a seed crystal, the silica sol may increase the production rate of the silica), the acidic silica sol is evenly dispersed in the entire reaction system, and the silica generated through the reaction of the sodium silicate and the sulfuric acid preferentially grows on the surface of the silica sol, to form the silica with an uniform and dispersed particle size. The sodium sulfate solution with a high concentration serves as an electrolyte and may accelerate the growth rate of the silica, to form the high-density, dense and uniform silica.

In step 3), firstly, a certain amount of sodium silicate solution is added to the reaction tank, after stopping adding the sodium silicate solution, the sulfuric acid solution is added at a certain speed until the pH value of the reaction solution is 5-7, this step is the reaction method that the acid is added to the sodium silicate, which can increase the growth rate of the silica while maintaining the dispersed state of the silica, to obtain the silica with the uniform particle size.

In step 4), adding the sulfuric acid solution with a high concentration to the reaction tank reduces a surface hydroxyl content of the silica, which further reduces the polymerization of the silica, ensures the uniformity of particle size and particle dispersion of the silica, and at the same time reduces the agglomeration of the finished silica product during the toothpaste preparation.

The beneficial effects of the present disclosure are as follows: the silica particle provided by the present disclosure has a small particle size, which can effectively block the dentinal tubules and microtubules on the tooth surface, isolate the channel of the external stimulation and hinder the transmission of excitement to a dental pulp nerve, thus alleviating the tooth sensitivity. Without chemical reactions, the silica particle will not cause a secondary damage to sensitive teeth, is safe and effective, has a long-lasting effect, and is suitable for preparing a toothpaste and other oral cleaning and care compositions.

Specifically:
1) The silica particle provided by the present disclosure solves the problem that the strontium salt, the fluoride and other remineralized anti-sensitivity products require a long time for the remineralization reaction, take effect slowly, and have potential safety hazards for special groups. The tooth sensitivity is relieved by complete physical blocking, with a fast action, no chemical reaction, high-efficiency and stability.
2) The silica particle provided by the present disclosure solves the problem that the potassium salt and Chinese herbal ingredients only paralyze tooth nerves and relieve sensitivity instantaneity and only relieve sensitivity and other issues by reducing sense of pain. By controlling the structural density of the product, the silica particle provided by the present disclosure is different to be fallen off in the dentin tubules, have a long-lasting effect, and effectively relieve the tooth sensitivity.
3) The silica particle provided by the present disclosure has a high apparent density, which can be quickly deposited during tooth brushing to directly block the exposed dental tubules, with an obvious effect. Moreover, the silica itself has stable chemical properties, is acid-resistant and salt-resistant, which will not cause any harm to the human body, and is a very safe blocking material when deposited in the tooth.
4) The silica particle provided by the present disclosure is used in the anti-sensitivity toothpaste, which may also provide a strong cleaning effect while relieving the tooth sensitivity, with dual effects of whitening teeth and relieving the tooth sensitivity.

### BRIEF DESCRIPTION OF DRAWINGS

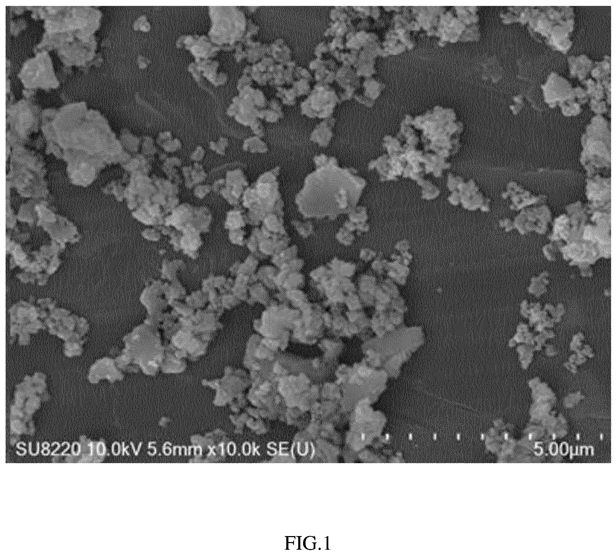

FIG. 1 is a Scanning Electron Microscope (SEM) image of a silica particle prepared in Example 1.

### DETAILED DESCRIPTION

The present disclosure will be further explained and described below in conjunction with specific examples.

### Example 1

A silica particle, which was prepared by a preparation method as follows:
1) 350kg of polyethylene glycol-400 was dissolved in 30m³ of water, a sodium silicate solution with a concentration of 0.5 mol/L and a sulfuric acid solution with a concentration of 1.5 mol/L were added simultaneously at a maintained flow rate, wherein the flow rate of the sodium silicate solution was 2 m³/h, and a pH value of a reaction solution during the reaction was controlled to 1; when an addition amount of the sodium silicate solution reached 12 m³, the sulfuric acid solution was stopped adding, the sodium silicate solution was continued to add until a pH value of a reaction solution was 3, and standing and aging were performed for 6 h to obtain acidic silica sol;
2) 200L of acidic silica sol with a mass fraction of 2% and 5m³ of sodium sulfate solution with a mass fraction of 20% were added to the reaction tank, then heated to 50°C, and the stirring was started at a rotation speed of 3500 r/min; the sodium silicate solution with a concentration of 0.75 mol/L and the sulfuric acid solution with a concentration of 4 mol/L were added simultaneously at a maintained flow rate, wherein the sodium silicate solution had a flow rate of 10 m³/h; and during the reaction, a pH value of a reaction solution was controlled to 7-8 and a temperature was controlled to 50 °C-60 °C for reaction for 30 min;
3) the sodium silicate solution and the sulfuric acid solution were stopped adding, heated to 60 °C, 10 m³ of sodium silicate solution with a concentration of 0.75 mol/L was added, the sulfuric acid solution with a concentration of 4 mol/L was added at a maintained flow rate of 3.6 m³/h until a pH value of a reaction solution was 6-7, and the sulfuric acid solution was stopped adding, allowed to stand and age for 25 min; and
4) the sulfuric acid solution with a volume fraction of 80% was added at a maintained flow rate of 2 m³/h until a pH value of a reaction solution was 4, the sulfuric acid solution was stopped adding, and press-filtering, washing, drying and crushing were performed to obtain the silica particle.

The scanning electron microscope (SEM) picture of the silica particle prepared in this example is as shown in FIG. 1.

It can be seen from FIG. 1 that the silica particle is in an irregular shape.

### Example 2

A silica particle, which was prepared by a preparation method as follows:
1) 350kg of polyethylene glycol-400 was dissolved in 30m³ of water, a sodium silicate solution with a concentration of 0.5 mol/L and a sulfuric acid solution with a concentration of 1.5 mol/L were added simultaneously at a maintained flow rate, wherein the flow rate of the sodium silicate solution was 2 m³/h, and a pH value of a reaction solution during the reaction was controlled to 1; when an addition amount of the sodium silicate solution reached 12 m³, the sulfuric acid solution was stopped adding, the sodium silicate solution was continued to add until a pH value of a reaction solution was 3, and standing and aging were performed for 6 h to obtain acidic silica sol;
2) 200L of acidic silica sol with a mass fraction of 2% and 5m³ of sodium sulfate solution with a mass fraction of 18% were added to the reaction tank, then heated to 50°C, and the stirring was started at a rotation speed of 3500 r/min; the sodium silicate solution with a concentration of 0.75 mol/L and the sulfuric acid solution with a concentration of 4 mol/L were added simultaneously at a maintained flow rate, wherein the sodium silicate solution had a flow rate of 10 m³/h; and during the reaction, a pH value of a reaction solution was controlled to 7-8 and a temperature was controlled to 50 °C-60 °C for reaction for 30 min;
3) the sodium silicate solution and the sulfuric acid solution were stopped adding, heated to 60 °C, 10 m³ of sodium silicate solution with a concentration of 0.75 mol/L was added, the sulfuric acid solution with a concentration of 4 mol/L was added at a maintained flow rate of 3.6 m³/h until a pH value of a reaction solution was 6-7, and the sulfuric acid solution was stopped adding, allowed to stand and age for 25 min; and
4) the sulfuric acid solution with a volume fraction of 80% was added at a maintained flow rate of 2 m³/h until a pH value of a reaction solution was 4, the sulfuric acid solution was stopped adding, and press-filtering, washing, drying and crushing were performed to obtain the silica particle.

After testing (same as Example 1), the silica particle prepared in this example is in an irregular shape.

### Example 3

A silica particle, which was prepared by a preparation method as follows:
1) 350kg of polyethylene glycol-400 was dissolved in 30m³ of water, a sodium silicate solution with a concentration of 0.5 mol/L and a sulfuric acid solution with a concentration of 1.5 mol/L were added simultaneously at a maintained flow rate, wherein the flow rate of the sodium silicate solution was 2 m³/h, and a pH value of a reaction solution during the reaction was controlled to 1; when an addition amount of the sodium silicate solution reached 12 m³, the sulfuric acid solution was stopped adding, the sodium silicate solution was continued to add until a pH value of a reaction solution was 3, and standing and aging were performed for 6 h to obtain acidic silica sol;
2) 200L of acidic silica sol with a mass fraction of 2% and 5m³ of sodium sulfate solution with a mass fraction of 15% were added to the reaction tank, then heated to 50°C, and the stirring was started at a rotation speed of 3500 r/min; the sodium silicate solution with a concentration of 0.75 mol/L and the sulfuric acid solution with a concentration of 4 mol/L were added simultaneously at a maintained flow rate, wherein the sodium silicate solution had a flow rate of 10 m³/h; and during the reaction, a pH value of a reaction solution was controlled to 8-9 and a temperature was controlled to 50 °C-60 °C for reaction for 30 min;
3) the sodium silicate solution and the sulfuric acid solution were stopped adding, heated to 60 °C, 10 m³ of sodium silicate solution with a concentration of 0.75 mol/L was added, the sulfuric acid solution with a concentration of 4 mol/L was added at a maintained flow rate of 3.6 m³/h until a pH value of a reaction solution was 6-7, and the sulfuric acid solution was stopped adding, allowed to stand and age for 25 min; and
4) the sulfuric acid solution with a volume fraction of 80% was added at a maintained flow rate of 2 m³/h until a pH value of a reaction solution was 4, the sulfuric acid solution was stopped adding, and press-filtering, washing, drying and crushing were performed to obtain the silica particle.

After testing (same as Example 1), the silica particle prepared in this example is in an irregular shape.

### Comparative Example 1

A silica particle, which was prepared by a preparation method as follows:
1) 350kg of polyethylene glycol-400 was dissolved in 30m³ of water, a sodium silicate solution with a concentration of 0.5 mol/L and a sulfuric acid solution with a concentration of 1.5 mol/L were added simultaneously at a maintained flow rate, wherein the flow rate of the sodium silicate solution was 2 m³/h, and a pH value of a reaction solution during the reaction was controlled to 1; when an addition amount of the sodium silicate solution reached 12 m³, the sulfuric acid solution was stopped adding, the sodium silicate solution was continued to add until a pH value of a reaction solution was 3, and standing and aging were performed for 6 h to obtain acidic silica sol;
2) 200L of acidic silica sol with a mass fraction of 2% and 5m³ of sodium sulfate solution with a mass fraction of 1% were added to the reaction tank, then heated to 50 °C, and the stirring was started at a rotation speed of 3500 r/min; the sodium silicate solution with a concentration of 0.75 mol/L and the sulfuric acid solution with a concentration of 4 mol/L were added simultaneously at a maintained flow rate, wherein the sodium silicate solution had a flow rate of 10 m³/h; and during the reaction, a pH value of the reaction solution was controlled to 7-8 and a temperature was controlled to 50°C-60 °C for reaction for 30 min;
3) the sodium silicate solution and the sulfuric acid solution were stopped adding, heated to 60 °C, 10 m³ of sodium silicate solution with a concentration of 0.75 mol/L was added, the sulfuric acid solution with a concentration of 4 mol/L was added at a maintained flow rate of 2 m³/h until a pH value of a reaction solution was 6-7, and the sulfuric acid solution was stopped adding, allowed to stand and age for 25 min; and
4) the aged product was heated to 85°C-100 °C, the sulfuric acid solution with a volume fraction of 80% was added at a maintained flow rate of 2 m³/h until a pH value of a reaction solution was 4, the sulfuric acid solution was stopped adding, and press-filtering, washing, drying and crushing were performed to obtain the silica particle.

### Comparative Example 2

A silica particle, which was prepared by a preparation method as follows:
1) 200 L of deionized water and 5 m³ of sodium sulfate solution with a mass fraction of 1% were added to the reaction tank, then heated to 50 °C, and the stirring was started at a controlled rotation speed of 3500 r/min; the sodium silicate solution with a concentration of 0.75 mol/L and the sulfuric acid solution with a concentration of 4 mol/L were added simultaneously at a maintained flow rate, wherein the sodium silicate solution had a flow rate of 10 m³/h; and during the reaction, a pH value of a reaction solution was controlled to 7-8 and a temperature was controlled to 50 °C-60 °C for reaction for 30 min;
2) the sodium silicate solution and the sulfuric acid solution were stopped adding, heated to 60 °C, 10 m³ of sodium silicate solution with a concentration of 0.75 mol/L was added, the sulfuric acid solution with a concentration of 4 mol/L was added at a maintained flow rate of 2 m³/h until a pH value of a reaction solution was 6-7, and the sulfuric acid solution was stopped adding, allowed to stand and age for 25 min; and
3) the aged product was heated to 85°C-100 °C, the sulfuric acid solution with a volume fraction of 80% was added at a maintained flow rate of 2 m³/h until a pH value of a reaction solution was 4, the sulfuric acid solution was stopped adding, and press-filtering, washing, drying and crushing were performed to obtain the silica particle.

### Performance testing:

1) The physical and chemical indexes of the silica particle prepared in Examples 1-3 and Comparative Examples 1-2 are as shown in the following table.

**Table 1 Physical and chemical indexes of silica particle**

| Physical and chemical indexs | Example 1 | | Example 2 | | Example 3 | | Comparative Example 1 | | Comparative Example 2 | |
|---|---|---|---|---|---|---|---|---|---|---|
| Water absorption capacity (mL/20g) | | 10.4 | | 10.8 | | 11.3 | | 15.0 | | 14.7 |
| Particle size D₁₀ (µm) | | 0.725 | | 0.852 | | 0.933 | | 2.993 | | 3.096 |
| Particle size D₃₀ (µm) | | 1.550 | | 1.787 | | 1.873 | | 6.998 | | 9.220 |
| Particle size D₅₀ (µm) | | 3.552 | | 3.773 | | 3.822 | | 25.324 | | 36.432 |
| Particle size D₇₅ (µm) | | 5.111 | | 5.308 | | 5.485 | | 45.330 | | 55.330 |
| Particle size D₉₀ (µm) | | 7.554 | | 8.003 | | 8.255 | | 60.229 | | 78.123 |
| Particle size D₉₇ (µm) | | 8.683 | | 8.893 | | 8.991 | | 80.223 | | 110.223 |
| Apparent density (g/mL) | | 0.47 | | 0.46 | | 0.45 | | 0.40 | | 0.51 |
| Copper loss (mg) | | 4.20 | | 5.09 | | 6.78 | | 9.20 | | 12.10 |
| Loss on Ignition (1000 °C) (%) | | 6.4 | | 6.2 | | 6.3 | | 6.0 | | 5.8 |
| RDA value | | 124 | | 130 | | 133 | | 180 | | 230 |
| PCR value | | 104 | | 102 | | 100 | | 108 | | 110 |
| Blocking rate (%) | | 86.46 | | 80.47 | | 78.37 | | 11.65 | | 8.57 |

| | | | | | | | | | | |
|---|---|---|---|---|---|---|---|---|---|---|
| Notes: Water absorption capacity: water absorption capacity was tested with reference to "QB/T 2346-2015 Oral Cavity Nursing Materials - Silicon Dioxide for Toothpaste". Particle sizes D₁₀, D₃₀, D₅₀, D₇₅, D₉₀ and D₉₇: particle sizes D₁₀, D₃₀, D₅₀, D₇₅, D₉₀ and D₉₇ were tested with reference to "GB/T 19077-2016 Particle Size Analysis - Laser Diffraction Method". Copper loss: copper loss was tested with reference to the method described in the paper "Research on the Copper Loss Value of Silica Used in Toothpaste". The test process were: washing the copper sheets with distilled water and then drying, weighing and recording the weight of the copper sheets, and then gently placing and fixing the copper sheets in the slot of the material pool; accurately dispersing different types of silica particles in the deionized water, stirring evenly and then pouring a resulting mixture into the material pool to immerse the copper sheets, after 10,000 times of friction, cleaning and drying the copper sheets, and weighing the difference of the copper sheets before and after friction to obtain the copper loss. Loss on Ignition (1000 °C): Loss on Ignition (1000 °C) was tested with reference to "QB/T 2346-2015 Oral Cavity Nursing Materials - Silicon Dioxide for Toothpaste". RDA value: RDA value was tested with reference to "ISO 11609:2017". PCR value: PCR value was tested with reference to "T/COCIA 7-2020 Efficacy Evaluation of Toothpaste - Laboratory Evaluation Method of Toothpaste to Remove Exogenous Stains". Blocking rate: blocking rate was tested with reference to the calculation method of tooth blocking rate in the group standard "T/COCIA 22-2023", the addition amount of slurry silica used was 15%. | | | | | | | | | | |

It can be seen from Table 1:
a) compared with Example 1, the concentration of the sodium sulfate solution was reduced in Examples 2 and 3, the particle size of the generated silica particle was larger, and the uniformity of the particle size decreased;
b) compared with Examples 1 and 2, the pH value of the process in Example 3 increased, the water absorption capacity of the generated silica particle was greater, the apparent density was reduced, and the blocking rate was slightly worse;
c) compared with Example 1, the post-acidification temperature was greatly increased in Comparative Example 1, and the concentration of the sodium sulfate solution was reduced at the same time, causing the particles to grow rapidly during the reaction and forming large agglomerated particles; and
d) no silica sol was added in Comparative Example 2, and the dispersed silica with an uniform particle size was not initially formed, the generated silica particle had a large and uneven particle size, and the friction force increased.

2) A toothpaste (toothpaste formula: 55wt% sorbitol, 20.4wt% distilled water, 1wt% carboxymethylcellulose, 5wt% thickening silica, 15wt% silica particle, 0.1wt% sodium benzoate, 0.2wt% sodium saccharin, 2wt% sodium lauryl sulfate, 1wt% essence and 0.3wt% titanium dioxide) was prepared with each of the silica particle prepared in Examples 1-3 and Comparative Examples 1-2 as an abrasive, and then a performance test was performed on the toothpaste, with the test results as shown in the following table.

**Table 2 Performance test results of toothpastes prepared with the silica particles prepared in Examples 1-3 and Comparative Examples 1-2**

| Performance indexs | Blocking rate (%) | (Copper loss (mg) | |
|---|---|---|---|
| Example 1 | 87.91 | | 4.5 |
| Example 2 | 83.45 | | 5.4 |
| Example 3 | 78.66 | | 5.8 |
| Comparative Example 1 | 30.20 | | 8.4 |
| Comparative Example 2 | 13.56 | | 10.6 |

| | | | |
|---|---|---|---|
| Notes: Blocking rate: blocking rate was tested with reference to the calculation method of tooth blocking rate in the group standard "T/COCIA 22-2023". Copper loss: the test process of copper loss was referred to the copper loss test method for the silica particle, but the test substance was changed to the toothpaste. | | | |

It can be seen from Table 2:
a) compared with Example 1, the friction value of the silica particle prepared in Examples 2 and 3 was larger, the friction value of the toothpaste produced was also larger, and its particle size distribution can still achieve the function of blocking dentin tubules, and the blocking rate was lower than that in Example 1; and
b) in Comparative Examples 1 and 2, the particle size of the silica particle was too large, the toothpaste produced had a poor blocking effect, and the friction value was high.

The above examples are preferred examples of the present disclosure, but the implementations of the present disclosure are not limited to the above examples. Any other changes, modifications, substitutions, combinations and simplifications made without departing from the gist and principles of the present disclosure should be equivalent substitutions, and are all included in the protection scope of the present disclosure.

## Claims

1. A silica particle, wherein the silica particle has (a particle size D₉₀- a particle size D₁₀)/ a particle size D₅₀=1.25-2.5, a particle size D₁₀<1 µm, a particle size D₃₀=1.5 µm-2 µm, a particle size D₅₀=3 µm-4 µm, a particle size D₇₅<5.5 µm, a particle size D₉₀=6 µm-8.5 µm, a particle size D₉₇<9 µm, and an apparent density of >0.3 g/mL.

2. The silica particle according to claim 1, wherein the silica particle has a particle size D₁₀>0.5 µm.

3. The silica particle according to claim 1, wherein the silica particle has an apparent density of >0.4 g/mL.

4. The silica particle according to any one of claims 1 to 3, wherein the silica particle has a copper loss of 2 mg-11 mg, a Radioactive Dentin Abrasivity (RDA) value <140, and a Pellicle Cleaning Ratio (PCR) value ≥100.

5. The silica particle according to any one of claims 1 to 3, wherein the silica particle has a water absorption capacity of <20 mL/20g.

6. The silica particle according to any one of claims 1 to 3, wherein a Loss on Ignition (1000 °C) of the silica particle is <8.5%.

7. A preparation method for the silica particle according to any one of claims 1 to 6, comprising the following steps:
1) dissolving polyethylene glycol in water, adding a sodium silicate solution and a sulfuric acid solution simultaneously at a maintained flow rate, and controlling a pH value of a reaction solution to 1-2 during reaction; stopping adding the sulfuric acid solution when an addition amount of the sodium silicate solution reaches 10 m³-15m³, and continuing to add the sodium silicate solution until a pH value of a reaction solution is 2-4, and performing aging to obtain acidic silica sol;
2) adding the acidic silica sol to a reaction tank, then adding a certain amount of sodium sulfate solution, heating to 50 °C to 60 °C and starting stirring; adding the sodium silicate solution and the sulfuric acid solution simultaneously at a maintained flow rate, and controlling a pH value of a reaction solution to 7-9 and a temperature to 50 °C-60 °C during reaction, and reacting for 20 min-40 min;
3) stopping adding the sodium silicate solution and the sulfuric acid solution, heating to 60 °C-70 °C, adding a certain amount of sodium silicate solution, adding the sulfuric acid solution at a maintained flow rate until a pH value of a reaction solution is 5-7, and stopping adding the sulfuric acid solution for aging; and
4) adding a sulfuric acid solution with a high concentration at a maintained flow rate until a pH value of a reaction solution is 3-5, stopping adding the sulfuric acid solution, and performing press-filtering, washing, drying and crushing to obtain the silica particle.

8. The preparation method according to claim 7, wherein an addition ratio of the polyethylene glycol to the water in step 1) is 300 kg-400 kg: 25 m³-35 m³.

9. The preparation method according to claim 7, wherein the sodium silicate solution in step 1) has a concentration of 0.2 mol/L-1 mol/L, the sodium silicate solution in step 1) has a flow rate of 1.5 m³/h-2.5 m³/h, the sulfuric acid solution in step 1) has a concentration of 1 mol/L-2 mol/L, and the aging in step 1) lasts for 5 h-7 h.

10. The preparation method according to claim 7, wherein the acidic silica sol in step 2) has a mass fraction of 1%-3%, the sodium sulfate solution in step 2) has a mass fraction of 15%-20%, the sodium silicate solution in step 2) has a concentration of 0.5 mol/L-1.25 mol/L, the sodium silicate solution in step 2) has a flow rate of 5 m³/h-12 m³/h, the sulfuric acid solution in step 2) has a concentration of 2 mol/L-6 mol/L, and the acidic silica sol, the sodium sulfate solution, and the sodium silicate solution in step 2) has a volume ratio of 1: 10-30: 10-30.

11. The preparation method according to claim 7, wherein the sodium silicate solution in step 3) has a concentration of 0.5 mol/L-1.25 mol/L, the sodium silicate solution in step 3) has an addition amount of 8 m³-12 m³, the sulfuric acid solution in step 3) has a flow rate of 2 m³/h-8 m³/h, and the aging in step 3) lasts for 10 min-45 min.

12. The preparation method according to claim 7, the sulfuric acid solution with a high concentration in step 4) has a volume fraction of 75%-85%, and the sulfuric acid solution with a high concentration in step 4) has a flow rate of 1 m³/h-4 m³/h.

13. An oral cleaning and care composition, comprising the silica particle according to any one of claims 1 to 6.

14. The oral cleaning and care composition according to claim 13, wherein the oral cleaning and care composition further comprises an orally acceptable excipient.

15. The oral cleaning and care composition according to claim 13 or 14, wherein the oral cleaning and care composition is a toothpaste.
